# FASCICULE DE BREVET EUROPEEN

(11) **EP 0 554 634 B1**
(45) Date de publication et mention de la délivrance du brevet: **27.03.1996**
(21) Numéro de dépôt: 92403362.4
(22) Date de dépôt: 11.12.1992
(51) Int. Cl.: A61K 38/20, A61K 47/36

(54) **Composition pharmaceutique stabilisée d'IL2 humaine recombinante non glycosylée sous forme réduite et son procédé de préparation**
Stabilisierte pharmaceutische Zusammensetzung die nicht-glycozylierte, reduzierte rekombinante human IL-2 umfasst und Verfahren zu ihrer Herstellung
Stabilized pharmaceutical composition comprising non-glycosylated, reduced, recombinant human IL-2 and process to prepare it

(30) Priorité: 12.12.1991 FR 9115418
(43) Date de publication de la demande: 11.08.1993
(73) Titulaire: ROUSSEL UCLAF, F-93230 Romainville (FR)
(72) Inventeur: Maneglier, Bruno, F-75020 Paris (FR); Voncken, Bernard, F-94570 Fosses sur Villiers (FR)
(74) Mandataire: Vieillefosse, Jean-Claude

(56) Documents cités:
- EP-A- 0 229 016
- EP-A- 0 251 001
- EP-A- 0 353 150
- EP-A- 0 452 598
- WO-A-89/09610
- WO-A-90/00397

## Description

La présente invention concerne une composition pharmaceutique d'interleukine 2 (noté IL2) humaine recombinante non glycosylée sous forme réduite qui a une stabilité améliorée, notamment sous forme lyophilisée, et qui convient à une utilisation comme médicament.

L'IL2 naturelle humaine qui est une lymphokine stimulant la prolifération de cellules T activées, possède 3 cystéines localisées en position 58, 105 et 125 de la séquence des 133 acides aminés de la protéine. Dans la molécule naturelle, les Cystéines 58 et 105 sont réunies par un pont disulfure, tandis que la cystéine 125 a un groupement sulfhydryle libre (Robb, R. J. et al. Proc. Natl. Acad. Sci. USA (1984) 81 6486-6490).

Des procédés de préparation de l'IL2 humaine, d'allèles ou de dérivés, par la technologie de l'ADN recombinant sont également décrits, par exemple dans le brevet européen EP 091539.

L'obtention de préparation d'IL2 naturelle ou recombinante de haute pureté, nécessaire à une utilisation comme médicament, conduit généralement à une instabilité de son activité biologique, notamment lors du stockage prolongé sous forme liquide, à l'état congelé ou sous forme lyophilisée.

Des stabilisants de l'activité biologique de l'IL2 pure sont connus et utilisés, par exemple des polyéthylène glycols, des composés réducteurs tels que le dithiothréitol et le mercaptoéthanol ou de l'albumine de sérum humain. Ainsi la demande de brevet européenne EP 0251001 décrit une composition d'IL2 naturelle pure dont l'activité biologique est stabilisée à la conservation prolongée en solution, à l'état congelé ou sous une forme lyophilisée par addition d'albumines ou de globulines, éventuellement combinées à d'autres agents tels que des sucres-alcools, des monomères ou des polymères de sucre tels que l'hydroxyamidon ou un Dextran.

Par ailleurs une nouvelle IL2 humaine recombinante, non glycosylée (notée r-hIL2), sous forme réduite, manifestant une activité biologique au moins égale à 0,5 x10⁷ U/mg et des compositions pharmaceutiques la renfermant, utilisables comme médicament, sont décrites dans la demande de brevet européen EP 0353150. Par IL2 sous forme réduite on entend une IL2 dont les 3 cystéines en position 58, 105 et 125 renferment un groupement sulfhydryle libre et manifestant une activité biologique comparable à celle de l'IL2 oxydée correspondante comportant le pont disulfure en position 58-105. Le dosage des groupements sulfhydryles, par exemple par spectrophotométrie avec la dithiopyridine, et le dosage classique de l'activité biologique, par mesure de la prolifération des lignées cellulaires murines CTLL-2, sont indiqués dans la demande de brevet EP 0353150.

Un exemple de composition pharmaceutique lyophilisée de la r-hIL2 réduite mentionnée ci-dessus, comprenant de l'acide citrique et du mannitol et convenant à une injection chez le malade est donné, ainsi qu'un exemple de composition liquide convenant plus particulièrement à une administration en perfusion continue.

D'une façon générale, la conservation prolongée de compositions pharmaceutiques comprenant une protéine pure comme principe actif et destinée à une utilisation comme médicament est une contrainte difficile à éviter. Mais un tel stockage est habituellement accompagné d'une dégradation qui conduit à une perte de l'activité biologique de la protéine, pour des raisons variées telles que des modifications physiques, par exemple la formation de dimère ou d'aggrégat ou telles que des modifications chimiques, par exemple une oxydation ou une désamidation. Aussi la stabilité de la composition contenant la protéine thérapeutique pendant des temps relativement longs, de préférence pendant un an, à une température convenable, de préférence à environ +5°C, est généralement exigée. L'obtention d'une telle stabilité nécessite toujours l'addition d'agents stabilisants qui diffèrent généralement selon la protéine concernée.

La demande de stabilité améliorée de compositions pharmaceutiques concerne notamment les compositions d'IL2 recombinante réduite ci-dessus et plus particulièrement les compositions de celle-ci sous forme liquide ou lyophilisée destinées à une administration chez le malade.

Les compositions liquides ou lyophilisées de r-hIL2 réduite ci-dessus qui sont décrites dans la demande de brevet EP 0353150 présente une stabilité convenable de l'activité biologique à la conservation, notamment en absence d'albumine, contrairement aux compositions d'IL2 naturelle ou recombinante connues qui renferment le pont disulfure en position 58-105 et qui présentent généralement une stabilité insuffisante de cette activité.

Cependant la formation de dimère de la r-hIL2 réduite ci-dessus peut être observée par électrophorèse sur gel de polyacrylamide (SDS-PAGE) notamment aux cours de la conservation à différentes températures des compositions lyophilisées, cependant sans être accompagnée d'une diminution significative de l'activité biologique manifestée. Par ailleurs, l'absence d'albumine comme agent de stabilisation dans les compositions de r-hIL2 réduite ci-dessus permet de suivre leur stabilité à l'aide d'une détection spectrophotométrique, par exemple à 280 nm, après une chromatographie liquide en phase inversée (noté RP-HPLC) qui met en évidence la formation éventuelle d'impuretés aux dépens de l'IL2, selon la durée et la température de conservation, cependant sans être accompagnée d'une diminution de l'activité biologique.

Bien que la formation de dimère ou la formation des impuretés détectées par RP-HPLC, dans le cas de la r-hIL2 réduite ci-dessus ne s'accompagnent pas d'une diminution de l'activité biologique telle que mesurée in vitro, de telles formations pourraient présenter par exemple l'inconvénient de permettre le développement de réactions antigéniques lors d'une administration prolongée chez le malade.

La présente invention concerne une nouvelle composition pharmaceutique stabilisée d'IL2 humaine recombinante non glycosylée sous forme réduite, caractérisée par l'absence d'albumine ou de tout autre agent absorbant en UV dans le domaine d'absorption des protéines ainsi que par l'absence essentielle de formation de dimère de l'IL2 au cours d'une conservation prolongée, notamment à l'état lyophilisé et qui convient à une utilisation comme médicament chez le malade. Par absence essentielle de formation de dimère, on entend que la teneur en dimère au cours de la conservation varie d'un pourcentage inférieur ou égal à 2 %.

De façon inattendue, l'amélioration de la stabilité des compositions de r-hIL2 réduite ci-dessus à la conservation, en particulier l'absence essentielle de formation de dimère de l'IL2 telle que définie ci-dessus, est obtenue par l'addition simultanée de Dextran et de sel monocalcique et disodique de l'acide éthylènediaminetétraacétique (noté EDTA calcique) à des solutions de r-hIL2 réduite comprenant de l'acide citrique et du mannitol.
Le Dextran est un polymère ramifié du glucose dont différentes fractions définissent des poids moléculaires (noté PM) moyens particuliers et sont utilisées notamment comme substitut du plasma sanguin en thérapeutique humaine. L'EDTA calcique est un agent chélatant convenant à un usage pharmaceutique.

L'objet de la présente demande concerne donc une composition pharmaceutique stabilisée d'IL2 recombinante humaine non glycosylée sous forme réduite comprenant de l'acide citrique et du mannitol et caractérisée en ce qu'elle comprend du Dextran et de l'EDTA calcique.

L'IL2 humaine recombinante, non glycosylée, sous forme réduite concernée par l'invention manifeste une activité biologique au moins égale à 0,5 x 10⁷ U/mg, de préférence égale à environ 1 x 10⁷ U/mg, selon le test de dosage indiqué ci-dessus. L'amélioration de la stabilité des compositions pharmaceutiques est observée à la conservation de compositions comprenant l'IL2 réduite définie ci-dessus, de l'acide citrique et du mannitol par addition d'un Dextran et d'EDTA calcique suivie de lyophilisation puis stockage pendant des temps prolongés à différentes températures. L'amélioration de la stabilité concerne la stabilité physique et la stabilité chimique de la r-hIL2 réduite qui peuvent être suivies à l'aide de méthodes analytiques, par exemple respectivement par SDS-PAGE et par RP-HPLC dont les conditions opératoires sont données plus loin.

L'invention concerne notamment une composition pharmaceutique d'IL2 recombinante humaine non glycosylée sous forme réduite caractérisée en ce que le Dextran a un PM moyen compris entre environ 40.000 et 110.000 et particulièrement en ce que le Dextran a un PM moyen d'environ 70.000.

On utilise les Dextran commercialisés, par exemple un Dextran 40, un Dextran 70 ou un Dextran 110 qui correspondent respectivement à des fractions de Dextran ayant des PM moyens d'environ 40.000, 70.000 et 110.000, de préférence un Dextran 70 auquel correspond un PM moyen d'environ 70.000.

L'invention concerne spécialement une composition pharmaceutique d'IL2 recombinante humaine non glycosylée sous forme réduite caractérisée en ce que le rapport en poids Dextran : IL2 est compris entre environ 20 et environ 80 et plus spécialement en ce que le rapport Dextran : IL2 est d'environ 40.

La masse de la r-hIL2 réduite est exprimé en mg de protéine mesurée de façon classique par la méthode de RP-HPLC. Par exemple, une composition lyophilisée renfermant 1 mg de r-hIL2 réduite ci-dessus est stabilisée par addition d'environ 20 à 80 mg de Dextran, de préférence environ 40 mg.

De préférence l'invention concerne une composition pharmaceutique d'IL2 humaine non glycosylée sous forme réduite caractérisée en ce que le rapport en poids IL2 : acide citrique : mannitol : Dextran : EDTA calcique est d'environ 1 : 10 : 50 : 40 : 0,01. Par exemple, une composition lyophilisée renfermant 1 mg de r-hIL2 réduite ci-dessus, 10 mg d'acide citrique et 50 mg de mannitol est stabilisée par addition d'environ 40 mg de Dextran et d'environ 10 microgrammes d'EDTA calcique.

La composition pharmaceutique stabilisée d'IL2 humaine recombinante non glycosylée sous forme réduite concernée par l'invention est caractérisée en ce qu'elle est essentiellement exempte de dimère de l'IL2. Par essentiellement exempte de dimère de l'IL2, on entend que la composition d'IL2 comprend une teneur en dimère de l'IL2 inférieure ou égale à 2 %.

L'invention concerne également un procédé de préparation de compositions pharmaceutiques lyophilisées stabilisées d'IL2 recombinante humaine non glycosylée sous forme réduite, caractérisé en ce que l'on ajoute une solution contenant un Dextran, de l'EDTA calcique et du mannitol à une solution aqueuse d'IL2 comprenant de l'acide citrique et lyophilise la solution ainsi obtenue. Un exemple de préparation est donné plus loin.

L'invention concerne aussi l'utilisation du Dextran et de l'EDTA calcique pour la stabilisation de compositions pharmaceutiques d'IL2 recombinante humaine non glycosylée sous forme réduite comprenant de l'acide citrique et du mannitol ainsi qu'une méthode de stabilisation de compositions pharmaceutiques d'IL2 recombinante humaine non glycosylée sous forme réduite comprenant de l'acide citrique et du mannitol caractérisé en ce que l'on ajoute du Dextran et de l'EDTA calcique.

Les exemples suivants illustrent l'invention sans toutefois la limiter.

### EXEMPLE 1: Composition pharmaceutique stabilisée de r-hIL2 réduite pour injection.

A 1210 ml d'une solution aqueuse contenant 2 mg/ml de r-hIL2 réduite et 18,2 mg/ml d'acide citrique obtenue par exemple comme décrit dans la demande de brevet EP 0353150 on ajoute 990 ml d'une solution aqueuse contenant 88 g de Dextran 70, 0,022 g d'EDTA calcique et 110 g de mannitol. La solution obtenue est filtrée sur une membrane de 0,22 microns de diamètre de pore. Après répartition stérile de 1ml de la solution homogène ainsi obtenue dans des flacons puis lyophilisation, les flacons-dose sont bouchés sous atmosphère d'azote et peuvent être conservés avant usage à une température de environ 4°C pendant au moins un an.

Les compositions lyophilisées ci-dessus ont la formule suivante :

| | |
|---|---|
| IL2 réduite | environ 1 mg |
| acide citrique | 10 mg |
| mannitol | 50 mg |
| Dextran 70 | 40 mg |
| EDTA calcique | 0,01 mg |

Ces compositions conviennent par exemple à une injection chez le malade après dissolution du contenu par injection de 1 ml d'eau distillée stérile, ainsi qu'à une perfusion continue par exemple après introduction de la solution reconstituée précédemment dans un conteneur Viaflex® contenant 500 ml de soluté pour perfusion Travenol® glucose à 5 %.

### EXEMPLE 2: stabilisation de la r-hIL2 réduite par le Dextran et l'EDTA calcique.

### 1 - Méthodes analytiques.

### a) Analytique par RP-HPLC :

La formation éventuelle d'impuretés pendant la conservation de compositions de r-hIL2 réduite ainsi que le dosage de l'IL2 réduite exprimée en mg de protéine sont estimés par RP-HPLC analytique sur une colonne C4 VYDAC (0,46 x 25 cm) 300 Å, 5 microns, au débit de 1 ml/mn, avec un gradient linéaire d'acétonitrile variant de 0 à 30 % sur 5 mn puis de 30 à 73 % sur 20 mn, contenant 0,1 % d'acide trifluoroacétique et avec une détection spectrophotométrique à 280 ou 220 nm.

### b) SDS-PAGE :

La formation éventuelle de dimère de l'IL2 est estimée par électrophorèse sur un gel de concentration à 20 % en acrylamide.

L'échantillon est chauffé au préalable pendant 2 mn à 100°C dans un tampon de dénaturation à 3 % de SDS et 5 % de mercaptoéthanol.

La migration est réalisée avec un tampon à 0,55 % de SDS et suivie d'une coloration à l'argent.

On détermine un PM apparent d'environ 15,5 Kda pour la r-hIL2 réduite et un PM apparent d'environ 31 Kda pour le dimère.

### c) activité biologique :

Elle est déterminée par mesure de la prolifération des lignées cellulaires leucémiques de souris CTLL-2 avec le test colorimétrique au tétrazolium (Mossmann T. et al., J. Immunol. Meth. (1983) 65 55-63).

### 2 - Stabilisation chimique de la r-hIL2 réduite par le Dextran et l'EDTA calcique.

La stabilisation chimique de la r-hIL2 par différents Dextran en combinaison avec l'EDTA calcique est étudié à la température de 37°C qui permet d'accélérer les éventuelles modifications chimiques de la protéine et de réaliser une étude sur des temps relativement courts.

L'étude est réalisée en partant d'une solution de r-hIL2 réduite contenant de l'acide citrique à laquelle on ajoute, sous atmosphère d'azote, une solution contenant de l'EDTA calcique, du mannitol et respectivement l'un des Dextran à étudier. On lyophilise la solution obtenue après répartition de 1 ml dans des flacons-dose que l'on maintient ensuite à 37°C pendant des temps variables sur une période d'un mois. Des flacons-témoin sans Dextran et sans EDTA calcique sont placés simultanément à 37°C. Le contenu de chaque flacon est ensuite repris par 1 ml d'eau distillée et analysé par RP-HPLC.

Les flacons-dose qui ont été soumis au test de stabilité à 37°C ont la formule suivante :

| | |
|---|---|
| IL2 réduite | 0,55 mg |
| acide citrique | 5 mg |
| mannitol | 50 mg |
| Dextran | 40 mg |
| EDTA calcique | 0,01 mg |

On a testé respectivement les Dextran T 40, T 70 et T 110 commercialisés par Pharmacia. Les résultats suivants ont été obtenus :

| Dextran | Conservation à 37°C | | | |
|---|---|---|---|---|
| | Pourcentage d'impuretés mesuré par RP-HPLC | | | |
| | t = 0 | t = 8j | t = 15j | t = 30j |
| - | 2,64 | n.i | n.i | n.i |
| Dextran 40 | 3,17 | 3,65 | 4,35 | 5,18 |
| Dextran 70 | 3,16 | 4,14 | 4,12 | 5,57 |
| Dextran 110 | 2,98 | 3,54 | 3,51 | 4,53 |

A la dose de 40 mg de Dextran, soit un rapport en poids IL2 : Dextran d'environ 80, les différents types de PM moyen des Dextran étudiés ont un effet comparable et remarquable sur la stabilité chimique de la r-hIL2 réduite à 37°C, alors qu'en absence de Dextran et d'EDTA calcique, la quantité d'impuretés formées rendent la RP-HPLC non interprétable (n.i) pour une période de conservation de seulement 8 jours.

L'influence de la concentration en Dextran 70 a été testée respectivement aux doses de 10 et 20 mg comparativement à la dose de 40 mg comme indiqué précédemment. Les résultats suivants ont été obtenus :

| Dextran 70 | Conservation à 37°C | |
|---|---|---|
| | Pourcentage d'impuretés mesuré par RP-HPLC | |
| | t = 0 | t = 30j |
| - | 2,64 | n.i |
| 10 mg | | 8,41 |
| 20 mg | | 5,81 |
| 40 mg | | 4,13 |

On observe un effet du Dextran sur la stabilité chimique de la r-hIL2 réduite, déjà notable à la plus faible dose qui correspond à un rapport en poids IL2 : Dextran d'environ 1:20.

### 3 - Stabilisation physique de la r-hIL2 réduite par le Dextran et l'EDTA calcique.

La stabilisation physique de la r-hIL2 réduite par le Dextran 70 en combinaison avec l'EDTA calcique est étudiée au cours de la conservation pendant des temps longs, compris entre un mois et un an, à différentes températures, respectivement à -20°C, +2/+8°C, température ambiante (environ 20° ± 5°C) et 37°C.

L'étude est réalisée en partant de la formule décrite à l'exemple 1. Des flacons-témoin sans Dextran et sans EDTA calcique sont placés simultanément dans les mêmes conditions de stockage. Le contenu de chaque flacon est repris par 1 ml de tampon de dénaturation indiqué ci-dessus et analysé par SDS-PAGE. On détermine le pourcentage de dimère.

On détermine également l'activité biologique sur des flacons ayant la même formulation avec Dextran. et EDTA calcique et conservés dans les mêmes conditions de température. Les résultats suivants ont été obtenus :

| Conservation | | IL2 dimère % (SDS-PAGE) | | 10⁷ UI/flacon formule Dextran/EDTA |
|---|---|---|---|---|
| Température °C | Durée (mois) | formule Dextran/EDTA | formule témoin | |
| - 20 | 1 | 1 | 1,5 | 1,15 |
| | 3 | 1 | 1 | |
| | 6 | 1 | 0,8 | 1,11 |
| | 9 | 1 | 1 | |
| | 12 | 1 à 2 | 1 | 1,18 |
| +2/+8 | 1 | 1 | 1,5 | 1,19 |
| | 3 | 1 | 1 | |
| | 6 | 1 | 0,8 | 1,18 |
| | 9 | 1 | 1 | |
| | 12 | 1 à 2 | 1 | 1,22 |
| +15/+25 | 1 | 1 | 1 | 1,12 |
| | 3 | 1 | 2 | |
| | 6 | 1 | 4 | 1,49 |
| | 9 | 1 | 10 | |
| | 12 | 1 à 2 | n.i | 1,28 |
| +37 | 1 | 1 | >10 | 1,27 |

On remarque que la formulation Dextran/EDTA empêche la formation de dimère de l'IL2 quelque soit la température étudiée, en particulier à la température ambiante et à 37°C pour lequelles la formulation témoin sans Dextran et sans EDTA montre une formation importante de dimère.

Par ailleurs, on confirme que la stabilisation physique obtenue par la formulation Dextran/EDTA permet également le maintien de la stabilité de l'activité biologique de l'IL2 réduite, quelle que soit la température de conservation.

La stabilisation obtenue selon le procédé de l'invention permet de réaliser une conservation prolongée des compositions de r-hIL2 réduite comprenant du Dextran et de l'EDTA calcique, conformément aux exigences d'une utilisation comme médicament.

## Revendications

1. Composition pharmaceutique stabilisée d'interleukine 2 recombinante humaine non glycosylée sous forme réduite comprenant de l'acide citrique et du mannitol et caractérisée en ce qu'elle comprend du Dextran et du sel monocalcique et disodique de l'acide éthylènediaminetétraacétique (EDTA calcique).

2. Composition selon la revendication 1 caractérisée en ce que le Dextran a un poids moléculaire moyen compris entre 40.000 et 110.000.

3. Composition selon la revendication 2 caractérisée en ce que le Dextran a un poids moléculaire moyen de 70.000.

4. Composition selon la revendication 3 caractérisée en ce que le rapport en poids Dextran : interleukine 2 est compris entre 20 et 80.

5. Composition selon la revendication 4 caractérisée en ce que le rapport Dextran : interleukine 2 est de 40.

6. Composition selon la revendication 5 caractérisée en ce que le rapport en poids interleukine 2 : acide citrique : mannitol : Dextran : EDTA calcique est de 1 : 10 : 50 : 40 : 0,01.

7. Composition selon l'une des revendications 1 à 6 caractérisée en ce qu'elle comprend une teneur en dimère de l'interleukine 2 inférieur ou égale à 2%.

8. Procédé de préparation de compositions pharmaceutiques lyophilisées stabilisées d'interleukine 2 recombinante humaine non glycosylée sous forme réduite, caractérisé en ce que l'on ajoute une solution contenant un Dextran, de l'EDTA calcique et du mannitol à une solution aqueuse d'interleukine 2 comprenant de l'acide citrique et lyophilise la solution ainsi obtenue.

9. Utilisation du Dextran et de l'EDTA calcique pour la stabilisation de compositions pharmaceutiques d'interleukine 2 recombinante humaine non glycosylée sous forme réduite comprenant de l'acide citrique et du mannitol.

10. Méthode de stabilisation de compositions pharmaceutiques d'interleukine 2 recombinante humaine non glycosylée sous forme réduite comprenant de l'acide citrique et du mannitol caractérisé en ce que l'on ajoute du Dextran et de l'EDTA calcique.

## Claims

1. Stabilized pharmaceutical composition of non-glycosylated recombinant human interleukin-2 in reduced form containing citric acid and mannitol and characterized in that it contains Dextran and a monocalcium and disodium salt of ethylenediaminetetraacetic acid (EDTA calcium).

2. Composition according to claim 1 characterized in that the Dextran has an average molecular weight comprised between 40,000 and 110,000.

3. Composition according to claim 2 characterized in that the Dextran has an average molecular weight of 70,000.

4. Composition according to claim 3 characterized in that the ratio by weight of Dextran : interleukin-2 is comprised between 20 and 80.

5. Composition according to claim 4 characterized in that the ratio of Dextran : interleukin-2 is 40.

6. Composition according to claim 5 characterized in that the ration by weight of interleukin-2 : citric acid : mannitol : Dextran : EDTA calcium is 1 : 10 : 50 : 40 : 0.01.

7. Composition according to one of claims 1 to 6 characterized in that it contains a level of interleukin-2 dimer of less than or equal to 2%.

8. Preparation process for stabilized, lyophilized pharmaceutical compositions of non-glycosylated recombinant human interleukin-2 in reduced form, characterized in that a solution containing a Dextran, EDTA calcium and mannitol is added to an aqueous solution of interleukin-2 containing citric acid and the solution thus obtained is lyophilized.

9. Use of Dextran and EDTA calcium for the stabilization of pharmaceutical compositions of non-glycosylated recombinant human interleukin-2 in reduced form containing citric acid and mannitol.

10. Stabilization method for pharmaceutical compositions of non-glycosylated recombinant human interleukin-2 in reduced form containing citric acid and mannitol characterized in that Dextran and EDTA calcium are added.

## Patentansprüche

1. Stabilisierte pharmazeutische Zusammensetzung von nichtglykosyliertem rekombinanten menschlichen Interleukin-2 in reduzierter Form, umfassend Zitronensäure und Mannit und gekennzeichnet dadurch, daß sie Dextran und Monocalciumsalz und Dinatriumsalz der Ethylendiamintetraessigsäure (Calcium-EDTA) enthält.

2. Zusammensetzung gemäß Anspruch 1, dadurch gekennzeichnet, daß das Dextran ein mittleres Molekulargewicht zwischen 40 000 und 110 000 hat.

3. Zusammensetzung gemäß Anspruch 2, dadurch gekennzeichnet, daß das Dextran ein mittleres Molekulargewicht von 70 000 hat.

4. Zusammensetzung gemäß Anspruch 3, dadurch gekennzeichnet, daß das Gewichtsverhältnis Dextran : Interleukin-2 zwischen 20 und 80 beträgt.

5. Zusammensetzung gemäß Anspruch 4, dadurch gekennzeichnet, daß das Verhältnis Dextran : Interleukin-2 40 ist.

6. Zusammensetzung gemäß Anspruch 5, dadurch gekennzeichnet, daß das Gewichtsverhältnis Interleukin-2 : Zitronensäure : Mannit : Dextran : Calcium-EDTA 1 : 10 : 50 : 40 : 0,01 ist.

7. Zusammensetzung gemäß einem der Ansprüche 1 bis 6, dadurch gekennzeichnet, daß sie einen Gehalt an Dimerem des Interleukins-2 unterhalb oder gleich 2 % enthält.

8. Verfahren zur Herstellung von stabilisierten lyophilisierten pharmazeutischen Zusammensetzungen von nichtglykosyliertem menschlichen rekombinanten Interleukin-2 in reduzierter Form, dadurch gekennzeichnet, daß man eine Lösung, die Dextran, Calcium-EDTA und Mannit enthält, zu einer wäßrigen Interleukin-2-Lösung gibt, die Zitronensäure enthält, und die so erhaltene Lösung lyophilisiert.

9. Verwendung von Dextran und Calcium-EDTA zur Stabilisierung von pharmazeutischen Zusammensetzungen von nichtglykosyliertem menschlichen rekombinanten Interleukin-2 in reduzierter Form, die Zitronensäure und Mannit enthalten.

10. Verfahren zur Stabilisierung von pharmazeutischen Zusammensetzungen von nichtglykosyliertem menschlichen rekombinanten Interleukin-2 in reduzierter Form, enthaltend Zitronensäure und Mannit, dadurch gekennzeichnet, daß man Dextran und Calcium-EDTA zugibt.
